# EUROPEAN PATENT SPECIFICATION

(11) **EP 1 903 024 B1**
(45) Date of publication and mention of the grant of the patent: **24.11.2010**
(21) Application number: 07253498.5
(22) Date of filing: 04.09.2007
(51) Int. Cl.: C07C 45/46, C07C 45/84, C07C 49/83, C08G 65/40

(54) **Improved process for the production of poly (ether ketone) - PEK- and its monomer**
Verbessertes Verfahren zur Herstellung von Poly(etherketon)-PEK und dessen Monomer
Procédé amélioré pour la production de poly(éther cétone) PEK et de son monomère

(30) Priority: 04.09.2006 IN MU14132006; 06.02.2007 GB 0702293
(43) Date of publication of application: 26.03.2008
(73) Proprietor: Gharda Chemicals Limited, Pin 421 203 (IN)
(72) Inventor: Gharda, Keki Horsmusji, Pin 421 203 (IN); Malte, Ashokkumar Maganlal, Pin 421 203 (IN); Mathur, Suchet Saran, Pin 421 203 (IN); Joseph, Pulinattu Cherian, Pin 421 203 (IN); Mathew, Abraham, Pin 421 203 (IN)
(74) Representative: Wise, Stephen James

(56) References cited:
- EP-A- 0 154 092
- EP-A- 0 344 688
- DATABASE CA [Online] CHEMICAL ABSTRACTS SERVICE, COLUMBUS, OHIO, US; MATSUMURA, SHUNICHI ET AL: "Preparation of high-purity 4-hydroxy-4'-halobenzophenone via 4-acetate" XP002465389 retrieved from STN Database accession no. 1991:408309 & JP 03 041047 A (TEIJIN LTD., JAPAN) 21 February 1991 (1991-02-21)
- DATABASE CA [Online] CHEMICAL ABSTRACTS SERVICE, COLUMBUS, OHIO, US; JIN, NING-REN ET AL: "A facile method for preparation of high- purity 4-hydroxy-4'-chlorobenzophenone" XP002465390 retrieved from STN Database accession no. 2001:460231 & ZHEJIANG GONGYE DAXUE XUEBAO , 28(2), 138-141 CODEN: ZDXUF2; ISSN: 1006-4303, 2000,

## Description

### Background :

Poly (Ether Ketone) - PEK - introduced by ICI Plc is a high performance polymer with a melting point (Tm) of 370°C and glass transition temperature (Tg) of 165°C. The polymer has good mechanical properties, high thermal properties, very good chemical resistance and oxidation stability. It finds application in aerospace, industrial and automotive applications due to its stability at elevated temperatures and in corrosive chemical environments. Though the low Tg results in a decrease in modulus at elevated temperatures, the superior thermal properties make it more acceptable in the high end applications.

In spite of its superior properties, commercial use of the polymer has so far been limited due to limited availability and high price. May be the need for higher processing temperatures was also one of the reasons for the limited use. Furthermore, the introduction of poly(ether ether ketone) - PEEK - by a more economically feasible route made inroads into applications where PEK was used. However, the need for an economic route for PEK remained a necessity and it is this objective and motivation, which have driven our research towards it.

Though several routes have been published in the literature on the synthesis of PEK, our research has shown that these are not economically feasible manufacturing processes for producing polymer meeting stringent quality requirements.

### SUMMARY OF THE INVENTION :

The present invention relates to a process firstly for producing high purity monomer 4-chloro-4'-hydroxy benzophenone (CHBP) by distillation under vacuum and then for the polymerization of the same to form PEK. Purification by vacuum distillation offers the high purity required for the monomer. The purification process is done by co-distilling with a solvent such as diphenylsulfone. Preferably the polymerization of the distillate is carried out in a mixture containing the same solvent.

In accordance with the present invention there is provided a process for the production of purified 4-chloro-4'-hydroxy benzophenone comprising distilling under reduced pressure a liquid containing 4-chloro-4'-hydroxy benzophenone and a compatible solvent which lowers the boiling point of the 4-chloro-4'-hydroxy benzophenone and is thermally stable at the lowered boiling point.

The present invention also provides a process for the production of unsubstituted poly ether ketone comprising the steps of (a) preparing purified 4-chloro-4'-hydroxy benzophenone by the process of the invention and then (b) polymerizing the thus purified 4-chloro-4'-hydroxy benzophenone.

### PUBLISHED LITERATURE ON PEK :

There are numerous papers and patents published on processes for PEK, based on both the nucleophilic and electrophilic routes. Some of the pertinent prior art is covered in the following publications, which pertain to the route starting from CHBP:
i) EP 0344688A2 describes an improved method of making the phenolate of CHBP,
ii) US Patent 4711945 describes polymerization in presence of a copper salt as the catalyst, and
iii) Polymer 1981, Vol. 22, page 1096, describes the preparation of PEK by reaction of 4,4'-difluoro diphenyl ketone and the potassium salt of 4,4'-dihydroxy diphenyl ketone in DPS02 solvent at 335°C. The same paper describes polymerization using, as the sole monomer, the potassium salt of 4-fluoro-4'-hydroxy diphenyl ketone, leading to branching of the polymer.

### DETAILED DESCRIPTION OF THE INVENTION :

Poly(ether ketone) - PEK - having the following repeating structure 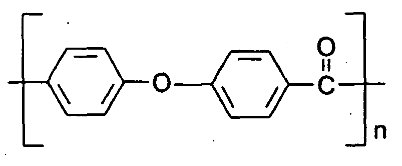 can be prepared by various methods described well in the literature. The prominent method described in the literature is by self condensation of the monomer 4-hydroxy-4'-fluoro or chlorobenzophenone. The fluoro derivative is more facile to react giving the polymer but has the disadvantage of higher cost and branching occurring during polymerization, resulting in inferior toughness property of the polymer. The chloro derivative is devoid of these problems.

The present inventors have concentrated on the use of 4-chloro-4'-hydroxy benzophenone as the monomer for polymerization to give PEK due to its ease of preparation and lower cost of production. However, our finding is that it is difficult to purify the monomer to the required purity level. Utilising repeated crystallizations after using adsorbants like silica and charcoal, monomer suitable for polymerization was obtained. However, the polymer prepared from such monomer lacked the properties required for withstanding severe thermal conditions. Our investigations in detail led us to believe that this is also a reason for the non-availability of the polymer at an economically affordable price.

Our research on this led to the finding that a good way to purify the monomer is by distillation under vacuum, which is hitherto unreported. 4-Chloro-4'-hydroxybenzophenone, during its manufacture retains high molecular weight impurities, aluminium salts, etc, which are difficult to be removed by normal purification methods. The physical properties of some relevant compounds are provided in Table1. Thermal instability, high melting point and boiling point pose problems in the distillation of CHBP.

However, using a short contact distillation technique and special scrape surface condensers, we succeeded in the distillation process. The narrow difference of boiling point and melting point made this method of distillation a highly skilled engineering operation. Compared with the monomer fomed via the crystallization route, the distilled product gave superior performance in the polymerization step and better polymer properties.

**TABLE 1**

| Compound | B.P. °C | Pressure mm Hg | M.P. °C | Thermal stability DSC method |
|---|---|---|---|---|
| 4-Chloro-4'- | 213 | 1 | 182 | Begins to |
| hydroxybenzophenone (CHBP) | 223 | 2 | | decompose at 240°C |
| Diphenylsulfone (DPSO2) | 195 | 2.5 | 129 | Stable up to |
| | 205 | 4.5 | | 378°C (B.P.) |
| Mixture of CHBP + DPS02 (1:2.6) | 192-205°C | 2.5 | 115-120 | - |

In order to overcome the difficulties in distillation, attempts were made to co-distill CHBP with other close boiling compounds. Reduced pressure distillation was tried and found effective for compatible solvents which lower the boiling point of CHBP and which are thermally stable at the lowered boiling point. Preferably the reduced pressure is less than 5mmHg, more preferably 3 to 1 mm Hg, especially about 2.5mm Hg. Due to diphenyl sulfone being used as a possible solvent for the polymerization step, this was tried as the compound for co-distillation with CHBP and it was surprisingly found that DPS02 is an abundantly suitable compound also for co-distillation.
Moreover, diphenyl sulfone is thermally very stable and is a very suitable solvent for the monomer for the polymerization step.
The compound chosen for co-distillation should be one having a boiling point close to that of 4-chloro-4'-hydroxy benzophenone and a lower melting point, and also should be thermally stable. Compounds other than DPS02 which can be used are diphenylene sulfone, benzophenone, dichloro benzophenone, etc. However, these others were not found to be as good as diphenyl sulfone for the co-distilling step.

The MP of the mixture of 1:2.6 parts of CHBP:DPS02, which is the optimum composition used for polymerization, dropped to 115°C. This solved the engineering problems associated with the high melting point during distillation of CHBP alone. Our experiments gave results which showed that the mixture of CHBP + DPS02 distilled at a lower temperature than the individual compounds and that the distillation proceeded smoothly. This was attributed possibly to an azeotrope formation between CHBP and DPS02. The generally preferred range for CHBP: co-distilling solvent is 1:2 to 1:5.

The polymerization is preferably carried out by forming the alkali metal salt of CHBP and conducting the polymerization in DPS02 solvent at 330°C. Whilst the alkali metal could be sodium (Na) or potassium (K), the preferred metal is K. The salt could be formed using alkali metal carbonate or hydroxide. The preferred base is K₂CO₃. Removal of water during the salt formation, whether K₂CO₃ or KOH was used, is important for the smooth polymerization process. The temperature required for reasonable rates of polymerization is in the range of 300-340°C, the more preferred range being 325-335°C.

The above invention of ours has led to an industrial process of purification of the key monomer CHBP suitable for polymerization, giving high quality PEK.

During our research work it was found that the selection of suitable materials of construction for the equipment for the polymerization step was important. Polymerization is done generally using alkali metal salts in DPS02 solvent at 330° C. Under these conditions significant corrosion occurs to stainless steel and the polymer gets contaminated with metals which are difficult to remove and cause an adverse effect on the polymer properties. The most suitable material of construction was found to be Hastelloy^{RTM}-C which experiences essentially no corrosion under the reaction conditions.

The present invention will now be explained below in further detail with examples of specific embodiments. However, it should be understood that the present invention is by no means restricted to the specific examples given below.

### Example 1

140 g of anhydrous AlCl₃ was slurried into 300 ml of o-dichlorobenzene (ODCB) solvent at 30°C. A solution of 94 g of phenol in 100 ml of ODCB was added over 2 hours at 30°C. The addition was exothermic and cooling was required. A solution of 174g of p-chlorobenzoyl chloride in 200 ml of ODCB was added to the mixture which was kept stirred at 30°C over 3 hours. After the addition, the mixture was heated to 80°C over 2 hours and maintained at 80°C . HCl gas was evolved and scrubbed out. Samples of the reaction mixture were analyzed by glc until >99% reaction was found to have been completed. The reaction mixture was drowned into 1 It of 0.5N HCl maintained at <30°C and then filtered. The cake was washed with water to remove most of the acidity. The wet cake was mixed into1 litre of water and basified to pH 11 by adding NaOH lye, and then filtered to remove the alkali insolubles. The filtrate which contains the sodium salt of the 4-chloro-4'-hydroxybenzophenone was carefully neutralized with dil. HCl until pH5 was reached. The product was filtered and washed with water and dried to give 198 g of solids. Yield : 85%.

The solid thus formed was further purified by dissolution in 1400 ml of a toluene-acetone (90:10) solvent mix at 80°C and charcoalised using 1 g of activated carbon. The filtrate was cooled to 20°C and filtered to give a product weighing 158 g, viz 68% overall yield. The product was analyzed by glc and was found to be >99% pure. However this product polymerized only with difficulty and did not give a polymer of good quality and consistency.

### Example 2

100 g of the product obtained in Example1 was taken for vacuum distillation for further purification to remove non-volatile substances. The apparatus consisted of a flask heated by the heating oil Therminol^{RTM}, an externally electrically heated vapor line, a solid scraping condenser, and a solids receiver. In an alterantive arrangement the vapor line could directly lead to the receiving pot which was kept cooled, and the product could then be removed by melting and casting into a tray.
The crude product was melted in the flask at at least 200°C and then a vacuum was applied to reduce the pressure in the flask to 1-2 mmHg. The distillation occurred in a vapor temperature range of 210-225°C. The distillation took about 1 hr. The distillate weight was 86g, i.e. 86% recovery, and its purity was found to be 99.5% by glc. It was a near white material. The product was directly used for polymerization.

### Example 3

100 g of the product obtained in Example 1 was mixed with 260 g of pure diphenyl sulfone in a 1000 ml distillation flask. The mixture was melted and distilled under vacuum directly at a vapor temperature of 192-205°C at a pressure of 2 mmHg. The product had a low melting point and a narrow boiling range. The distilled product was analyzed by glc analysis. The analysis indicated that 96 g of CHBP had distilled out along with the DPS02 solvent. The distillation residue weighed 3.5g indicating low degradation during distillation. The distilled product along with the co-distilled DPS02 was used for polymerization.

### Example 4

116.3 g of the distilled CHBP obtained from Example 2 was mixed with 300 g of diphenyl sulfone in a polymerization reactor. The reactor consisted of a 3" dia Hastelloy^{RTM}-C cylindrical vessel with an oil heating jacket arrangement. The stirrer was of a helical type made of the same Hastelloy^{RTM}-C material. The stirrer motor was provided with a sensor arrangement for measuring the torque developed during the polymerization reaction and to indicate the viscosity level of the reaction mixture. A thermocouple temperature indicator was present in the reactor to measure the reaction mixture temperature. Provision for N₂ gas purging of the reactor was also made.

To the reaction mixture, 0.2625 moles of K₂CO₃ (36.30 g) passable through a 200 mesh sieve was added at 135°C. The mixture was heated to 200°C and maintained for one hour under an N₂ atmosphere to facilitate the dehydration of the mixture. The temperature was then increased to 300°C over 1.5 hrs and maintained for 2 hrs. The temperature was then further raised to 330°C over 1 hr and maintained at this temperature to build the required viscosity of the reaction mixture. When the required viscosity was reached, the polymer was end capped by adding 1 mole% of 4-fluoro benzophenone and maintaining for 0.5 hrs.
The reaction mixture was cooled to 300°C and discharged into another vessel containing 2 It of chlorobenzene solvent kept stirred at 130°C. The mixture was filtered at 130° and the cake refluxed with chlorobenzene and filtered. The process was repeated 4 or 5 times until all the DPS02 solvent was leached out. The cake was further refluxed with water to remove mineral salts and dried to give 93 g of solids - a yield of 95% PEK polymer. The solids as a 0.25% solution in 98% H₂SO₄ had an inherent viscosity of 0.92 dl/g.

### Example 5

The experimental procedure of Example 4 was repeated with the exception that the distillate mixture of CHBP and DPS02 obtained from Example 3 was directly used after analyzing the content of CHBP in it. The batch size was adjusted to 0.5 mole (116.3 g) CHBP as in Example 4 and the process was continued in the same way.
The weight of product obtained was 94 g, i.e. a weight yield of 96%. The inherent viscosity measured as a 0.25% solution in 98% H₂SO₄ was 0.93 dl/g.

## Claims

1. A process for the production of purified 4-chloro-4'-hydroxy benzophenone comprising distilling under reduced pressure a liquid containing 4-chloro-4'-hydroxy benzophenone and a compatible solvent which lowers the boiling point of the 4-chloro-4'-hydroxy benzophenone and is thermally stable at the lowered boiling point.

2. A process as claimed in claim 1 wherein the compatible solvent is diphenyl sulphone.

3. A process as claimed in claim 2 wherein the ratio in the liquid being distilled of 4-chloro-4'-hydroxy benzophenone to diphenyl sulphone is between 1:2 and 1:5 by volume.

4. A process as claimed in claim 3 wherein the said ratio is 1:2.6 by volume.

5. A process for the production of unsubstituted poly ether ketone comprising the steps of (a) preparing purified 4-chloro-4'-hydroxy benzophenone by a process as claimed in any one of the preceding claims, and then (b) polymerising the thus purified 4-chloro-4'-hydroxy benzophenone.

6. A process as claimed in claim 5 wherein the purified benzophenone is polymerised in the presence of diphenyl sulphone.

7. A process as claimed in claim 5 or claim 6 wherein the polymerization is carried out at a temperature of 330°C.

## Patentansprüche

1. Ein Verfahren zur Produktion von gereinigtem 4-Chlor-4'-hydroxy-benzophenon, das das Destillieren einer Flüssigkeit, die 4-Chlor-4'-hydroxy-benzophenon und ein kompatibles Lösungsmittel, das den Siedepunkt des 4-Chlor-4'-hydroxy-benzophenons senkt und bei dem gesenkten Siedepunkt thermisch stabil ist, enthält, bei reduziertem Druck beinhaltet.

2. Verfahren gemäß Anspruch 1, wobei das kompatible Lösungsmittel Diphenylsulfon ist.

3. Verfahren gemäß Anspruch 2, wobei das Volumenverhältnis von 4-Chlor-4'-hydroxy-benzophenon zu Diphenylsulfon in der destillierten Flüssigkeit zwischen 1:2 und 1:5 liegt.

4. Verfahren gemäß Anspruch 3, wobei das Volumenverhältnis 1:2,6 beträgt.

5. Ein Verfahren zur Produktion von nicht substituiertem Polyetherketon, das die folgenden Schritte beinhaltet: (a) Zubereiten von gereinigtem 4-Chlor-4'-hydroxybenzophenon durch ein Verfahren gemäß einem der vorhergehenden Ansprüche und dann (b) Polymerisieren des derartig gereinigten 4-Chlor-4'-hydroxy-benzophenons.

6. Verfahren gemäß Anspruch 5, wobei das gereinigte Benzophenon in Gegenwart von Diphenylsulfon polymerisiert wird.

7. Verfahren gemäß Anspruch 5 oder Anspruch 6, wobei die Polymerisation bei einer Temperatur von 330 °C durchgeführt wird.

## Revendications

1. Un procédé de production de benzophénone 4-chloro-4'-hydroxy purifiée comprenant la distillation sous pression réduite d'un liquide contenant de la benzophénone 4-chloro-4'-hydroxy et d'un solvant compatible qui abaisse le point d'ébullition de la benzophénone 4-chloro-4'-hydroxy et est thermiquement stable au point d'ébullition abaissé.

2. Un procédé tel que revendiqué dans la revendication 1 où le solvant compatible est la diphénylsulfone.

3. Un procédé tel que revendiqué dans la revendication 2 où le rapport benzophénone 4-chloro-4'-hydroxy sur diphénylsulfone dans le liquide qui est distillé se trouve entre 1/2 et 1/5 en volume.

4. Un procédé tel que revendiqué dans la revendication 3 où ledit rapport est de 1/2,6 en volume.

5. Un procédé de production de polyéthercétone non substitué comprenant les étapes consistant à (a) préparer de la benzophénone 4-chloro-4'-hydroxy purifiée par un procédé tel que revendiqué dans l'une quelconque des revendications précédentes, puis (b) polymériser la benzophénone 4-chloro-4'-hydroxy ainsi purifiée.

6. Un procédé tel que revendiqué dans la revendication 5 où la benzophénone purifiée est polymérisée en présence de diphénylsulfone.

7. Un procédé tel que revendiqué dans la revendication 5 ou la revendication 6 où la polymérisation est effectuée à une température de 330 °C.
